(19) Europäisches Patentamt
European Patent Office
Office européen des brevets

(11) EP 0 728 754 B1

(12) **EUROPÄISCHE PATENTSCHRIFT**

(45) Veröffentlichungstag und Bekanntmachung des Hinweises auf die Patenterteilung:
**28.11.2001 Patentblatt 2001/48**

(51) Int Cl.[7]: **C07D 311/16**

(21) Anmeldenummer: **96101798.5**

(22) Anmeldetag: **08.02.1996**

(54) **Cumarinderivate, Verfahren zur Herstellung und ihre Verwendung als Zwischenprodukte**

Coumarin derivatives, method for their preparation and their use as intermediates

Dérivés de coumarine, procédé de préparation et leur utilisation comme intermédiaires

(84) Benannte Vertragsstaaten:
**AT BE CH DE FR GB IT LI NL**

(30) Priorität: **21.02.1995 DE 19505940**

(43) Veröffentlichungstag der Anmeldung:
**28.08.1996 Patentblatt 1996/35**

(73) Patentinhaber: **BAYER AG**
**51368 Leverkusen (DE)**

(72) Erfinder:
- **Chen, Yun, Dr.**
**D-47800 Krefeld (DE)**
- **Wehrmann, Rolf, Dr.**
**D-47800 Krefeld (DE)**
- **Köhler, Burkhard, Dr.**
**D-51373 Leverkusen (DE)**

(56) Entgegenhaltungen:
**US-A- 5 286 803**

- **PATENT ABSTRACTS OF JAPAN vol. 17, no. 119 (C-1034), 12.März 1993 & JP-A-43 000991 (MITSUI TOATSU)**
- **CHEMICAL ABSTRACTS, vol. 120, no. 24, 13.Juni 1994 Columbus, Ohio, US; abstract no. 310930u, Seite 840; XP002004148 & JP-A-05 263 072 (MITSUI TOATSU)**
- **PATENT ABSTRACTS OF JAPAN vol. 12, no. 230 (C-508), 29.Juni 1988 & JP-A-63 023901 (NIPPON KAYAKU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 728 754 B1

## Beschreibung

Die Erfindung betrifft Cumarin-Derivate der Formel (I)

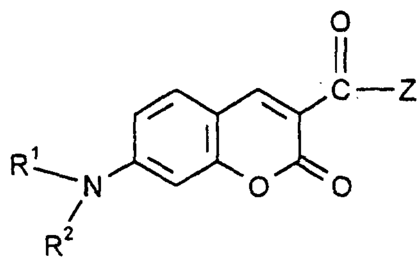

(I)

wobei

$R^1$ und $R^2$ unabhängig voneinander für gegebenenfalls durch $C_1$-$C_4$-Alkoxycarbonyl substituiertes $C_1$-$C_{16}$-Alkyl, jeweils unsubstituiertes oder durch $C_1$-$C_4$-Alkyl, $C_1$-$C_4$-Alkoxycarbonyl, Chlor und/oder Brom substituiertes Phenyl, Naphthyl, Phenyl-$C_1$-$C_4$-alkyl oder Naphthyl-$C_1$-$C_4$-alkyl stehen.

$R^1$ und $R^2$ stehen insbesondere für gegebenenfalls durch Hydroxy, Amino oder Carboxy substituiertes $C_1$-$C_6$-Alkyl oder Phenyl,

Z steht für $OR^3$ oder $NR^4R^5$, wobei $R^3$ für $C_1$-$C_{16}$-Alkyl, Phenyl, Naphthyl, Phenyl-$C_1$-$C_4$-alkyl oder Naphthyl-$C_1$-$C_4$-alkyl steht, die jeweils durch mindestens eine Hydroxygruppe substituiert sind, und wobei die aromatischen Ringe zusätzlich noch durch Halogen, $C_1$-$C_6$-Alkyl, $C_1$-$C_6$-Alkoxy substituiert sein können,

$R^4$ und $R^5$ stehen unabhängig voneinander für jeweils gegebenenfalls durch Hydroxy substituiertes $C_1$-$C_{16}$-Alkyl, Phenyl, Naphthyl, Phenyl-$C_1$-$C_4$-alkyl oder Naphthyl-$C_1$-$C_4$-alkyl steht, wobei einer der Reste $R^4$ oder $R^5$ über eine Hydroxygruppe verfügt und die aromatischen Ringe zusätzlich noch durch Halogen, $C_1$-$C_6$-Alkyl, $C_1$-$C_6$-Alkoxy substituiert sein können.

$R^3$ steht besonders bevorzugt für durch Hydroxy substituiertes $C_1$-$C_{12}$-Alkyl.

$R^4$ und $R^5$ stehen unabhängig voneinander besonders bevorzugt für gegebenenfalls durch Hydroxy substituiertes $C_1$-$C_{12}$-Alkyl, wobei mindestens einer der Reste $R^4$ und $R^5$ über eine Hydroxygruppe verfügt, wobei die aliphatischen Kohlenstoffketten, wie z.B. Alkyl, Alkoxy, Alkylamino, Aralkyl, in $R^1$, $R^2$, $R^3$, $R^4$ und $R^5$ durch eines oder mehrere, vorzugsweise ein oder zwei Heteroatome ausgewählt aus Sauerstoff, Stickstoff und Schwefel und/oder durch ein oder mehrere, vorzugsweise ein oder zwei Phenylenringe, die durch $C_1$-$C_4$-Alkyl und/oder Halogen substituiert sein können, unterbrochen sein können.

Die erfindungsgemäßen Cumarin-Derivate der Formel (I) tragen mindestens eine Hydroxygruppe über die sie an Polymerseitenketten chemisch gebunden werden können.

Die erfindungsgemäßen Verbindungen eignen sich daher zur Herstellung von Polymeren mit Luminophoren an den Seitenketten. Solche Polymere können in der lichtemittierenden Schicht einer Leuchtdiode eingesetzt werden.

Die Erfindung betrifft weiterhin ein Verfahren zur Herstellung von Cumarin-derivaten der Formel (I),

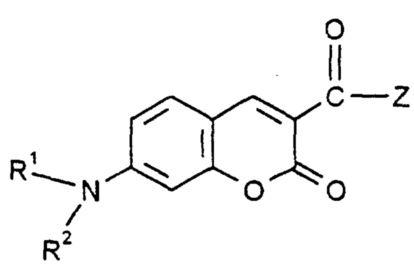

(I)

wobei $R^1$, $R^2$ und Z die oben genannte Bedeutung haben, dadurch gekennzeichnet, daß man

a) im Falle, daß Z für $-OR^3$ steht, vorzugsweise in einem Eintopfverfahren, aus der Meldrum's-Säure der Formel (II)

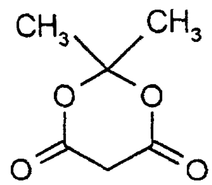

(II)

und einem Alkohol der Formel (III)

$R^3$-OH (III)

gegebenenfalls in Gegenwart eines Verdünnungsmittels wie beispielsweise Toluol, Xylol oder Mesitylen unter Katalyse von beispielsweise p-Toluolsulfonsäure bei Temperaturen im Bereich von 20 bis 250°C, vorzugsweise 80 bis 150°C, das Malonsäure-Derivat der Formel (IV) herstellt,

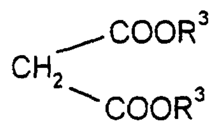

(IV)

und dieses anschließend mit einem Salicylaldehyd der Formel (V)

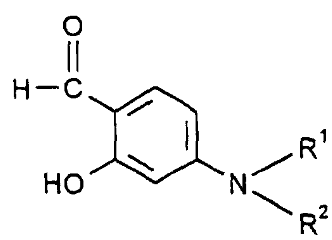

(V)

wobei $R^1$, $R^2$, $R^3$ die o.g. Bedeutung haben
gegebenenfalls in Gegenwart eines Verdünnungsmittels wie beispielsweise Toluol, Xylol oder Mesitylen unter Katalyse von beispielsweise Piperidinacetat bei Temperaturen von 50 bis 250°C, vorzugsweise 80 bis 140°C umsetzt, und
b) im Falle, daß Z für

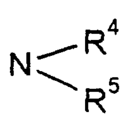

steht, einen Salicylaldehyd der Formel (V) ein sekundäres Amin der Formel (VI) und ein Malonsäure-Derivat der Formel (VII)

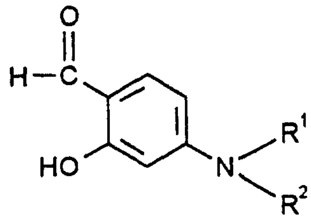

(V)

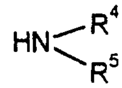

(VI)

$CH_2(COOR^6)_2$

(VII)

worin

| | |
|---|---|
| $R^1$, $R^2$, $R^4$ und $R^5$ | die oben genannte Bedeutung haben und |
| $R^6$ | für $C_1$-$C_6$-Alkyl steht, |

gegebenenfalls in Gegenwart eines Verdünnungsmittels wie beispielsweise Toluol, Xylol oder Mesitylen unter Katalyse von beispielsweise Piperidinacetat bei Temperaturen von 50 bis 250°C, vorzugsweise 80 bis 140°C umsetzt.

Bei der Durchführung des erfindungsgemäßen Verfahrens a) setzt man im allgemeinen auf 1 Mol Meldrum's Säure der Formel (II) 2-10 Mol, vorzugsweise 3-6 Mol Alkohol der Formel (III) und pro Mol Malonsäure-Derivat der Formel (IV) setzt man im allgemeinen 0,5-1,0, vorzugsweise 0,9-1,0 Mol Salicylaldehyd der Formel (V) ein.

Bei der Durchführung des erfindungsgemäßen Verfahrens b) setzt man pro Mol Salicylaldehyd der Formel (V) im allgemeinen 2-20, vorzugsweise 5-10 Mol sekundäres Amin und 1-2, vorzugsweise 1,2-1,5 Mol Malonsäure-Derivat der Formel (VII) ein.

Die Herstellung der erfindungsgemäßen Curmarinderivate der Formel (I) für Z = $OR^3$ im Sinne einer Knoevenagel-Kondensation und anschließender Cyclisierung wird beispielhaft durch das folgende Formelschema dargestellt:

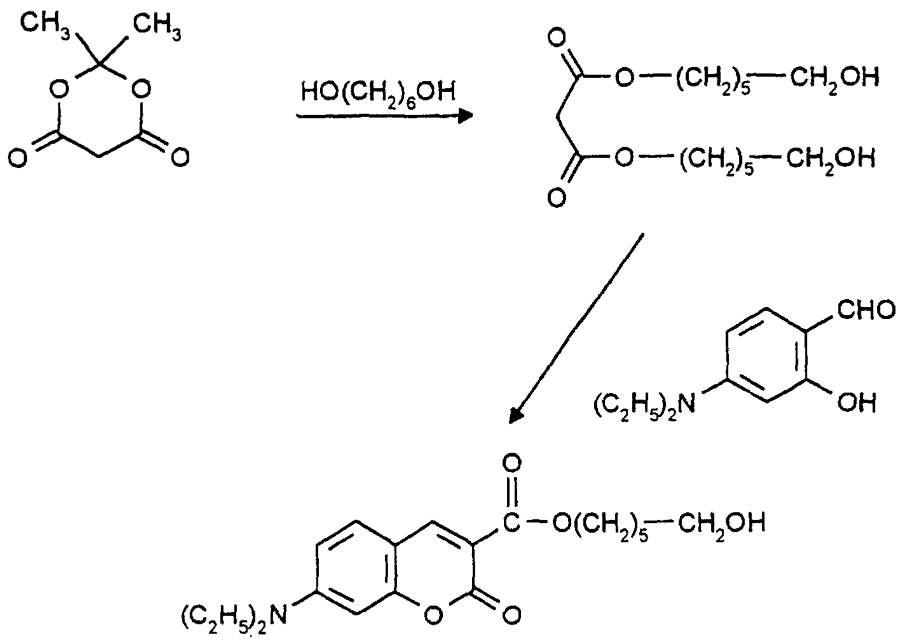


Hierbei wird zunächst Bis-(6-hydroxyhexyl)-malonat durch Umsetzung von der Meldrum's-Säure und 1,6-Hexandiol in Gegenwart katalytischer Mengen von p-Toluolsulfonsäure unter Aceton- und Wasseraustritt hergestellt. Anschließend versetzt man das Bis-(6-hydroxyhexyl)-malonat mit 4-Diethylaminosalicyladehyd in Gegenwart katalytischer Mengen Piperidinacetat unter Bildung des erwünschten 3-(6-Hydroxyhexoxycarbonyl)-7-diethylamino-cumarins.

Die Herstellung der erfindungsgemäßen Cumarinderivate der Formel (I) für Z = $NR^4R^5$ wird beispielhaft durch

das folgende Formelschema dargestellt:

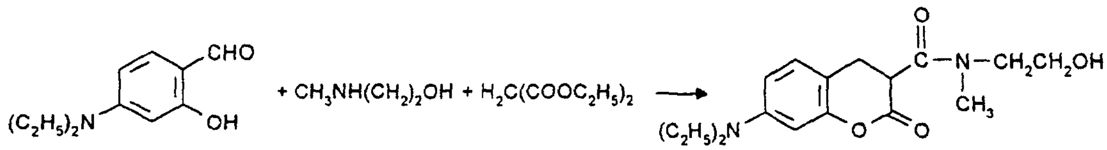

Hierbei wird 4-Diethylamino-salicylaldehyd in Gegenwart katalytischer Mengen Piperidinacetat mit Diethylmalonat und 2-(Methylamino)-ethanol umgesetzt. Man erhält das erwünschte 3-[(N-Hydroxyethyl-N-methyl)aminocarbonyl]-7-diethylamino-cumarin.

Die Ausgangsprodukte der Formeln (II), (III), (IV), (V), (VI) und (VII) sind allgemein bekannte Verbindungen der organischen Chemie.

Die erfindungsgemäßen Verbindungen der Formel (I) eignen sich zur Herstellung von (Co)Polymeren mit Luminophoren an den Seitenketten, die zum Aufbau von elektrolumineszierenden Anordnungen geeignet sind. Die (Co)Polymere sind aufgebaut aus mindestens einem wiederkehrenden Kettenbaustein der allgemeinen Formel (1) oder (2) und gegebenenfalls wiederkehrende Einheiten der allgemeinen Formel (3)

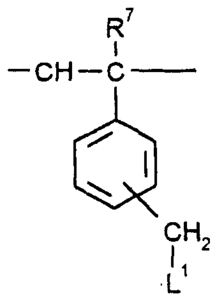

(1)

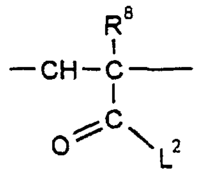

(2)

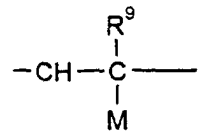

(3)

worin

| | |
|---|---|
| $R^7$, $R^8$ und $R^9$ | unabhängig voneinander Wasserstoff oder $C_1$-$C_6$-Alkyl bedeuten, |
| M | für CN oder $C_1$-$C_{30}$-Alkoxycarbonyl, $C_1$-$C_{30}$-(Di)Alkylaminocarbonyl, $C_1$-$C_{30}$-Alkylcarbonyl steht, die jeweils durch Hydroxy oder $C_1$-$C_6$-Alkoxycarbonyl substituiert sein können, weiterhin für Phenyl, Naphthyl, Anthracenyl, Pyridyl oder Carbazolyl steht, die jeweils durch Reste aus der Gruppe Halogen, Hydroxy, Silyl, $C_1$-$C_{30}$-Alkyl, $C_6$-$C_{18}$-Aryl, $C_1$-$C_{30}$-Alkoxy, $C_1$-$C_{30}$-Alkoxycarbonyl, $C_1$-$C_{30}$-Acyloxy und $C_1$-$C_{30}$-Alkylcarbonyl substituiert sein können, |
| $L^1$ und $L^2$ | unabhängig voneinander für oben beschriebene Derivate der Formel (I) stehen, wobei die Verknüpfung an die Methylengruppe am Phenyl des Monomers (1) bzw. die Carbonylgruppe des Monomer (2) über das Sauerstoff der OH-Gruppe am Cumarin-Derivat erfolgt durch Reaktion der OH-Gruppe mit einer reaktiven Gruppe, z.B. Halogen, (z.B. bei Monomer (1) $-CH_2Cl$, bei Monomer (2) -COCl). |

Der Anteil der Struktureinheiten der Formel (3) beträgt 0 bis 99,5, vorzugsweise 40 bis 99,5 Mol-% und der Anteil an Struktureinheiten (1) oder (2) jeweils 0,5 bis 100, vorzugsweise 0,5 bis 60 Mol-%, wobei sich die Molanteile zu 100 % ergänzen.

Die oben beschriebenen (Co)Polymere lassen sich durch übliche Polymerisationsverfahren aus den entsprechenden Monomeren der Formeln (4) und (5) und gegebenenfalls (6) herstellen:

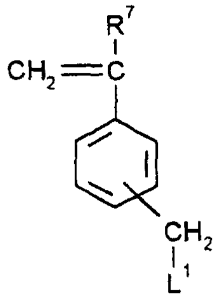

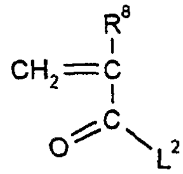

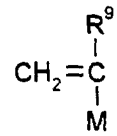

(4) (5) (6)

Die Monomere der Formeln (4) und (5) werden erhalten aus einem mit mindestens einer OH-Gruppe funktionalisiertem Cumarin-Derivat der Formel (I) und einem Styrol- oder Acrylsäure-Derivat der Formeln (7) oder (8)

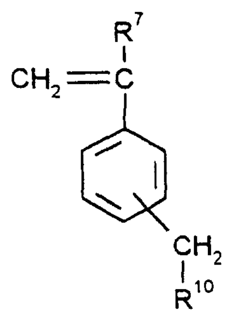

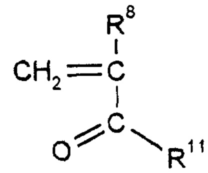

(7) (8)

worin

$R^{10}$ für Halogen, vorzugsweise Cl oder Brom steht und

$R^{11}$ für Halogen, vorzugsweise Cl oder Brom, Hydroxy oder $C_1$-$C_4$-Alkoxy steht,

in Gegenwart einer Base wie Triethylamin, Pyridin oder ein AlkalimetallAlkoholat bei Temperaturen von -30°C bis 100°C, vorzugsweise 0°C bis 60°C umsetzt.

Die Monomere der Formel (6) sind bekannt bzw. lassen sich nach bekannten Verfahren herstellen.

Die Polymerisationsverfahren sind in der Literatur beschrieben. Sie können ionisch oder radikalisch erfolgen. Eine anionische Polymerisation kann z.B. durch Starter wie Butyllithium oder Lithiumnaphthalid ausgelöst werden. Eine radikalische Polymerisation kann z.B. durch Radikalstarter wie z.B. Azoinitiatoren oder Peroxide, vorzugsweise AIBN (Azoisobuttersäuredinitril) oder Dibenzoylperoxid, ausgelöst werden. Die Herstellung der Polymere kann in Substanz oder in geeigneten Lösungsmitteln wie Benzol, Toluol; Tetrahydrofuran, Dioxan, Ethylacetat, Xylol, Chlorbenzol, 1-Methoxy-2-propylacetat, chlorierte Kohlenwasserstoffe, Aceton, etc., bei Temperaturen von 20-250°C erfolgen.

Die Styrol- und Acrylsäure-Derivate der Formeln (7) und (8) und die Monomere der Formel (6) sind allgemein bekannte Verbindungen.

Die Verwendung der erfindungsgemäßen Cumarin-Derivate der Formel (I) zur Herstellung von (Co)Polymerisaten mit Luminopheren an den Seitenketten wird beispielsweise durch das folgende Formelschema dargestellt:

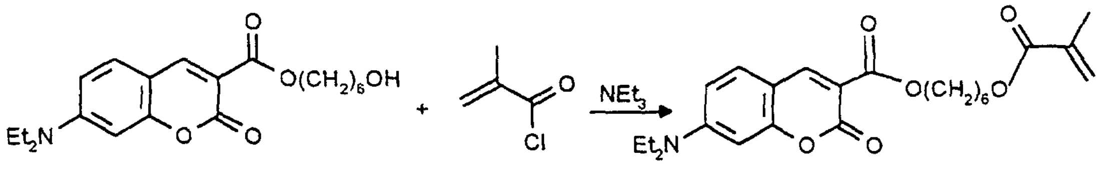


(9) (10) (11)

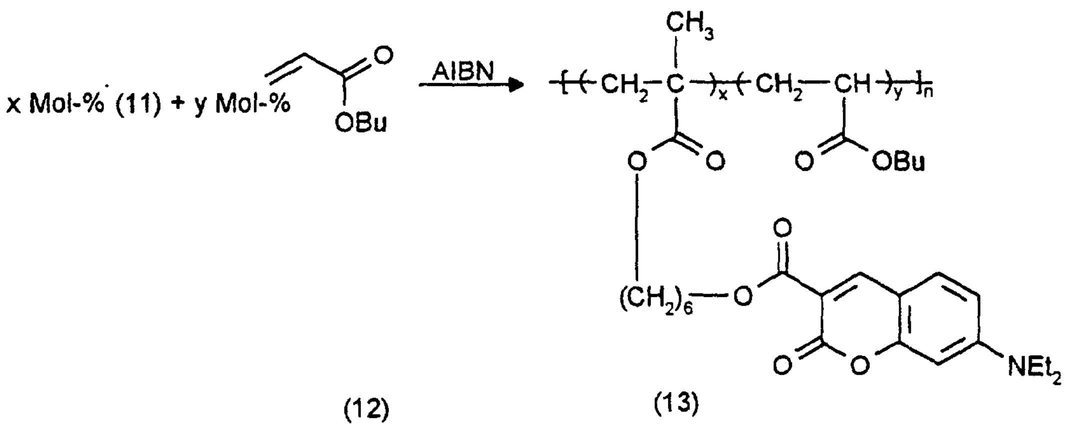


(12) (13)

Hierbei wird ausgehend von 3-(6-Hydroxyhexoxycarbonyl)-7-diethylaminocumarin (9) und Methacryloylchlorid (10) unter Mitwirkung von Triethylamin bei 0°C bis Raumtemperatur zunächst das Methacrylat (11) hergestellt. Das Methacrylat (11) läßt sich in Gegenwart von n-Butylacrylat (12) als Comonomer unter Mitwirkung von AIBN als Radikalstarter in Chlorbenzol bei 100°C unter Bildung des Copolymerisats (13) polymerisieren.

Die (Co)Polymerisate weisen Molekulargewichte ($\overline{M}w$) im Bereich von 500 bis 1 Million g/Mol, vorzugsweise 800 bis 500.000 g/mol, bestimmt durch Gelpermeationschromatographie, auf. Sie zeichnen sich durch ihre lumineszierenden Eigenschaften und Filmbildungsvermögen aus und lassen sich durch Gießen, Rakeln oder Spin-Coating auf geeignete Unterlagen aufbringen. Die Produkte zeigen sowohl in Lösungen als auch als Filme Photolumineszenz bei Bestrahlung. Die (Co)Polymerisate sind zum Aufbau von elektrolumineszierenden Anzeigen geeignet. Diese sind dadurch gekennzeichnet, daß sich eine elektrolumineszierende Schicht zwischen zwei Elektroden befindet, daß. mindestens eine der beiden Elektroden im sichtbaren Spektralbereich transparent ist,
daß beim Anlegen einer Gleichspannung im Bereich von 0,1 bis 100 Volt Licht im Frequenzbereich von 200 bis 2000 nm emittiert wird,
daß zwischen der elektrolumineszierenden Schicht und den Elektroden zusätzlich eine oder mehrere Zwischenschichten angeordnet sein können.

Diese Zwischenschichten sind aus der Literatur bekannt (vergl. Appl. Phys. Lett., 57, 531 (1990)) und werden dort als HTL (hole transport layer) und als ETL (electron transport layer) bezeichnet. Der Zweck solcher Zwischenschichten liegt u.a. in der Erhöhung der Elektrolumineszenzintensität.

Die erfindungsgemäßen elektrolumineszierenden Polymere können auch als eine Mischung miteinander oder mit mindestens einem weiteren Material in der elektrolumineszierenden Schicht eingesetzt werden. Bei diesem weiteren Material kann es sich um inerte Binder, ladungsträgertransportierende Substanzen wie in EP-A 532 798 oder EP-A 564 224 beschrieben, oder Mischungen aus inerten Bindern und ladungsträgertransportierenden Substanzen handeln.

Die Mischungen aus den erfindungsgemäßen Polymeren und einem weiteren Material zeichnen sich u.a. dadurch aus, daß sie filmbildend sind und durch Gießen, Rakeln oder Spin-coating großflächig auf geeignete Substrate aufgebracht werden können. Als Substrat sind transparente Träger wie Glas oder Kunststoff-Folien (z.B. Polyester, wie Polyethylenterephthalat. oder Polyethylennaphthalat, Polycarbonat, Polysulfon, Polyimid-Folien) geeignet.

Bei dem inerten Binder handelt es sich vorzugsweise um lösliche transparente Polymere wie z.B. Polycarbonate, Polystyrol, Polyvinylpyridin, Polymethylphenylsiloxan und Copolymere des Polystyrols wie SAN, Polysulfone,

Polyacrylate, Polyvinylcarbazol, Vinylacetat- und Vinylalkoholpolymere und -copolymere.

## Beispiele

### Beispiel 1

Eine Lösung von Bis-(6-hydroxyhexyl)-malonat in 1,6-Hexandiol wird beim Erhitzen einer Mischung aus 21,6 g (0,15 mol) Meldrum's-Säure, 59 g (0,50 mol), 1,6-Hexandiol und 0,28 g (1,5 mmol) p-Toluolsulfonsäuremonohydrat für 2 h bei 140°C dargestellt.

Anschließend versetzt man die resultierende Lösung mit 26,0 g (0,135 mol) 4-Diethylamino-salicylaldehyd, 0,7 ml Piperidin und 0,1 ml Essigsäure. Die Reaktionsmischung wird 3 h bei 110°C gerührt und nach Abkühlung mit 300 ml Wasser versetzt. Man extrahiert die Suspension mit Dichlormethan. Die organische Phase wird eingeengt und der Rückstand aus Toluol umkristallisiert.

Man erhält 40,2 g (83 % der Theorie) 3-(6-Hydroxyhexoxycarbonyl)-7-diethylamino-cumarin als gelbe Kristalle mit einem Schmelzpunkt von 85 bis 86°C.

### Beispiel 2

Unter Rühren versetzt man eine Mischung aus 19,3 g (0,10 mol) 4-Diethylaminosalicylaldehyd und 19,2 g (0,12 mol) Diethylmalonat mit 30 ml 2-(Methylamino)-ethanol, 0,3 ml Piperidin und 0,3 ml Essigsäure. Die Reaktionsmischung wird 4 h bei 100°C gerührt und nach Abkühlung mit 200 ml Wasser versetzt. Man extrahiert die Suspension mit Dichlormethan. Die organische Phase wird eingeengt und der Rückstand aus Toluol umkristallisert.

Man erhält 25,4 g (80 % der Theorie) 3-[(N-Hydroxyethyl-N-methyl)-aminocarbonyl]-7-diethylamino-cumarin als braune Kristalle mit einem Schmelzpunkt von 94,5 bis 96,5°C.

### Verwendungsbeispiel

1. Herstellung des 3-(6-Methacryloxyhexoxycarbonyl)-7-diethylamino-cumarin, Formel (11) im Reaktionsschema, Beispiel 1

   Zu einer Lösung von 16,3 g (0,045 Mol) 3-(6-Hydroxyhexoxycarbonyl)-7-diethylamino-cumarin (9) und 10,0 g (0,10 Mol) frisch destilliertem Triethylamin in 50 ml trockenem Tetrahydrofuran tropft man unter Rühren und Eiswasser-Kühlung 8,36 g (0,08 Mol) Methacryloylchlorid (10). Die Reaktionsmischung wird 5 h bei Raumtemperatur gerührt. Anschließend wird die Reaktionsmischung mit 200 ml Wasser und 200 ml Methylenchlorid behandelt. Nach der Phasentrennung wird die wäßrige Phase noch zweimal mit je 100 ml Methylenchlorid extrahiert. Die vereinigten organischen Auszüge werden neutral gewaschen und mit Natriumsulfat getrocknet. Nach Abdestillieren des Lösungsmittels im Vakuum wird der Rückstand durch eine kurze Kieselgelsäule mit Diethylether als Laufmittel adsorptiv filtriert. Nach Abdestillieren des Lösungsmittels erhält man 17,6 g (92 % der Theorie) blaßgelbes Öl.

2. Herstellung des Copolymerisats gemäß Formel (13) im Reaktionsschema mit x = 13 Mol-%, y = 87 Mol-%

   Eine Lösung von 5,0 g (0,012 Mol) 3-(6-Methacryloxyhexoxycarbonyl)-7-diethylamino-cumarin (11), 10,0 g (0,078 Mol) n-Butylacrylat (12) und 0,15 g (0,91 mMol) AIBN in 80 ml trockenem Chlorbenzol wird unter Vakuum entgast und anschließend unter Stickstoff bei 100°C 3 h gerührt. Die Polymerisationsmischung wird dann mit 0,15 g (0,91 mMol) AIBN in drei Portionen innerhalb 3 h nachgestartet. Die Lösung wird anschließend in 100 ml Methanol unter Rühren zugetropft und dann die Suspension abgesaugt. Das Rohprodukt wird noch zweimal aus Methylenchlorid/Methanol-Mischung gefällt. Ausbeute 12,7 g (85 % der Theorie).

3. Herstellung einer elektrolumineszierenden Anordnung

ITO beschichtetes Glas (hergestellt von der Firma Balzers) wird in 20 x 30 mm große Substrate geschnitten und gereinigt. Dabei werden folgende Schritte sukzessiv durchgeführt:

1) 15 min mit dest. Wasser und Falterol im Ultraschallbad spülen,
2) 2 x 15 min mit jeweils frischem dest. Wasser im Ultraschallbad spülen,
3) 15 min mit Ethanol im Ultraschallbad spülen,
4) 2 x 15 min mit jeweils frischem Aceton im Ultraschallbad spülen,
5) Trocknen auf fusselfreien Linsentüchern.

Eine 1 %ige Lösung vom Polymer gemäß Formel (40) (Beispiel 4) in 1,2-Dichlorethan wird gefiltert (0,2 µm-Filter, Firma Sartorius). Die gefilterte Lösung wird mit einer Lackschleuder bei 1000 U/min auf dem ITO-Glas verteilt. Die Dicke des trockenen Films beträgt 110 nm und der Ra-Wert der Oberfläche beträgt 5 nm (Stylusprofilometer Alpha-Step 200 der Firma Tencor Inst.).

Der so hergestellte Film wird anschließend mit Al-Elektroden bedampft. Dazu werden mit Hilfe einer Lochmaske isolierte Al-Punkte mit einem Durchmesser von 3 mm auf den Film gedampft. Während des Aufdampfens herrscht in einer Aufdampfapparatur (Leybold) ein Druck unter $10^{-5}$ mbar.

Die ITO-Schicht und die Al-Elektrode wird über elektrische Zuführung mit einer Spannungsquelle verbunden. Beim Erhöhen der Spannung fließt ein elektrischer Strom durch die Anordnung und die beschriebene Schicht elektrolumineszierte im blauen Spektralbereich.

## Patentansprüche

1. Cumarin-Derivate der Formel (I)

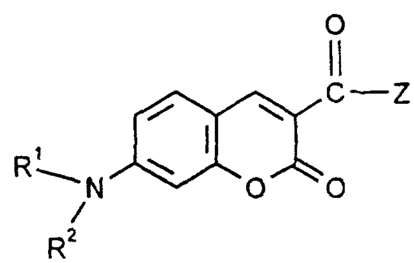

(I)

wobei

| | |
|---|---|
| $R^1$ und $R^2$ | unabhängig voneinander für gegebenenfalls durch $C_1$-$C_4$-Alkoxycarbonyl substituiertes $C_1$-$C_{16}$-Alkyl, jeweils unsubstituiertes oder durch $C_1$-$C_4$-Alkyl, $C_1$-$C_4$-Alkoxycarbonyl, Chlor und/oder Brom substituiertes Phenyl, Naphthyl, Phenyl-$C_1$-$C_4$-alkyl oder Naphthyl-$C_1$-$C_4$-alkyl stehen, oder |
| Z | für $OR^3$ oder $NR^4R^5$ steht, wobei $R^3$ für $C_1$-$C_{16}$-Alkyl, Phenyl, Naphthyl, Phenyl-$C_1$-$C_4$-alkyl oder Naphthyl-$C_1$-$C_4$-alkyl steht, die jeweils durch mindestens eine Hydroxygruppe substituiert sind, und wobei die aromatischen Ringe zusätzlich noch durch Halogen, $C_1C_6$-Alkyl, $C_1$-$C_6$-Alkoxy substituiert sein können, |
| $R^4$ und $R^5$ | unabhängig voneinander für jeweils gegebenenfalls durch Hydroxy substituiertes $C_1$-$C_{16}$-Alkyl, Phenyl, Naphthyl, Phenyl-$C_1C_4$-alkyl oder Naphthyl-$C_1$-$C_4$-alkyl stehen, wobei einer der Reste $R^4$ oder $R^5$ über eine Hydroxygruppe verfügt und die aromatischen Ringe zusätzlich noch durch Halogen, $C_1$-$C_6$-Alkyl, $C_1$-$C_6$-Alkoxy substituiert sein können, wobei die aliphatischen Kohlenstoffketten, wie z.B. Alkyl, Alkoxy, Alkylamino, Aralkyl, in $R^1$, $R^2$, $R^3$, $R^4$ und $R^5$ durch eines oder mehrere, vorzugsweise ein oder zwei Heteroatome ausgewählt aus Sauerstoff, Stickstoff und Schwefel und/oder durch ein oder mehrere, vorzugsweise ein oder zwei Phenylenringe, die durch $C_1$-$C_4$-Alkyl und/oder Halogen substituiert sein können, unterbrochen sein können. |

2. Cumarin-Derivate der Formel (I) gemäß Anspruch 1, **dadurch gekennzeichnet, daß**

| | |
|---|---|
| $R^1$ und $R^2$ | für gegebenenfalls durch Hydroxy, Amino oder Carboxy substituiertes $C_1$-$C_6$-Alkyl oder Phenyl stehen, oder |
| $R^1$ und $R^2$ | gemeinsam mit dem Stickstoffatom, an das sie gebunden sind, einen Morpholin-, Piperidin-, Pyrrolidin- oder Piperazinring steht, der einen oder zwei Substituenten aus der Gruppe Methyl, Ethyl und Phenyl tragen kann, |

$R^3$ für durch Hydroxy substituiertes $C_1$-$C_{12}$-Alkyl,

$R^4$ und $R^5$ unabhängig voneinander für gegebenenfalls durch Hydroxy substituiertes $C_1$-$C_{12}$-Alkyl stehen, wobei mindestens einer der Reste $R^4$ und $R^5$ über eine Hydroxygruppe verfügt.

**3.** Verfahren zur Herstellung von Cumarinderivaten der Formel (I) gemäß Anspruch 1
wobei man

a) im Falle, daß Z für -$OR^3$ steht, aus der Meldrum's-Säure der Formel (II)

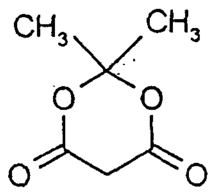

(II)

und einem Alkohol der Formel (III)

$$R^3\text{-OH} \quad \text{(III)}$$

gegebenenfalls in Gegenwart eines Verdünnungsmittels unter Katalyse bei Temperaturen im Bereich von 20 bis 250°C das Malonsäure-Derivat der Formel (IV) herstellt,

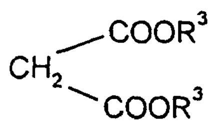

(IV)

und dieses anschließend mit einem Salicylaldehyd der Formel (V)

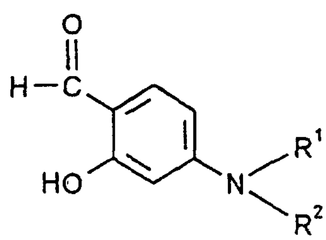

(V)

wobei $R^1$, $R^2$, $R^3$ die in Anspruch 1 genannte Bedeutung haben
gegebenenfalls in Gegenwart eines Verdünnungsmittels unter Katalyse bei Temperaturen von 50 bis 250°C umsetzt, und
b) im Falle, daß Z für

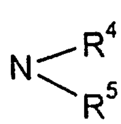

steht, einen Salicylaldehyd der Formel (V) ein sekundäres Amin der Formel (VI) und ein Malonsäure-Derivat der Formel (VII)

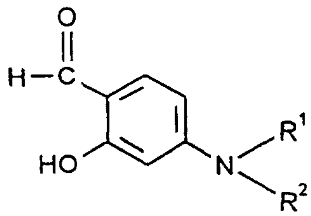

(V)

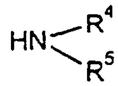

(VI)

$CH_2(COOR^6)_2$

(VII)

worin

| | |
|---|---|
| $R^1$, $R^2$, $R^4$ und $R^5$ | die oben genannte Bedeutung haben und |
| $R^6$ | für $C_1$-$C_6$-Alkyl steht, |

gegebenenfalls in Gegenwart eines Verdünnungsmittels bei Temperaturen von 50 bis 250°C umsetzt.

**4.** Verwendung von Cumarin-Derivaten der Formel (I) gemäß Anspruch 1 zur Herstellung von Polymeren mit Luminophoren an den Seitenketten.

**5.** Cumarin - Derivate der Formel (I) gemäß Anspruch 1:

3-(6-Hydroxyhexoxycarbonyl)-7-diethylamino-cumarin,
3-[(N-Hydroxyethyl-N-methyl)-amino-carbonyl]-7-diethylamino-cumarin.

## Claims

**1.** Coumarin derivatives of formula (I)

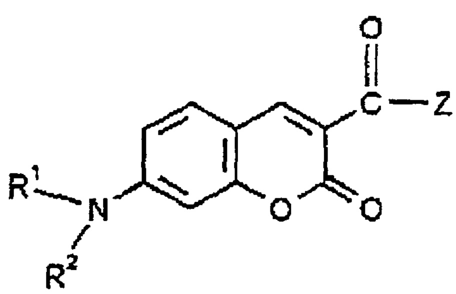

(I)

wherein

| | |
|---|---|
| $R^1$ and $R^2$ | represent, independently of each other, a $C_1$-$C_{16}$ alkyl which is optionally substituted by a $C_1$-$C_4$ alkoxycarbonyl, or a phenyl, napththyl, phenyl-$C_1$-$C_4$ alkyl or napththyl-$C_1$-$C_4$ alkyl, each of which is unsubstituted or is substituted by a $C_1$-$C_4$ alkyl, a $C_1$-$C_4$ alkoxycarbonyl, chlorine and/or bromine, or |
| Z | represents $OR^3$ or $NR^4R^5$, wherein $R^3$ represents a $C_1$-$C_{16}$ alkyl, phenyl, napththyl, a phenyl-$C_1$-$C_4$ alkyl or a napththyl-$C_1$-$C_4$ alkyl, each of which is substituted by at least one hydroxy group, and wherein the aromatic rings may also be substituted in addition by a halogen, a $C_1$-$C_6$ alkyl or a $C_1$-$C_6$ alkoxy, |
| $R^4$ and $R^5$, | independently of each other, represent a $C_1$-$C_{16}$ alkyl, phenyl, napththyl, a phenyl-$C_1$-$C_4$ alkyl or a napththyl-$C_1$-$C_4$ alkyl, which are each optionally substituted by hydroxy, wherein one of the $R^4$ or |

$R^5$ radicals contains a hydroxy group and wherein the aromatic rings can also be substituted in addition by a halogen, a $C_1$-$C_6$ alkyl or a $C_1$-$C_6$ alkoxy, wherein the aliphatic carbon chains, such as alkyl, alkoxy, alkylamino or aralkyl, for example, in $R^1$, $R^2$, $R^3$, $R^4$ and $R^5$ can be interrupted by one or more, preferably one or two, heteroatoms selected from oxygen, nitrogen and sulphur and/or by one or more, preferably one or two, phenylene rings which can be substituted by a $C_1$-$C_4$ alkyl and/or by a halogen.

**2.** Coumarin derivatives of formula (I) according to claim 1, **characterised in that**

$R^1$ and $R^2$ represent a $C_1$-$C_6$ alkyl or phenyl which is optionally substituted by hydroxy, amino or carboxy, or

$R^1$ and $R^2$, jointly with the nitrogen atom to which they are bonded, represent a morpholine, piperidine, pyrrolidine or piperazine ring which can contain one or two substituents from the group comprising methyl, ethyl and phenyl,

$R^3$ represents a $C_1$-$C_{12}$ alkyl which is substituted by hydroxy, and

$R^4$ and $R^5$, independently of each other, represent a $C_1$-$C_{12}$ alkyl which is optionally substituted by hydroxy, wherein at least one of the $R^4$ and $R^5$ radicals contains a hydroxy group.

**3.** A method of preparing coumarin derivatives of formula (I) according to claim 1
wherein

a) when Z represents -$OR^3$, the malonic acid derivative of formula (IV)

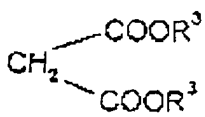

(IV)

is prepared from the Meldrum's acid of formula (II)

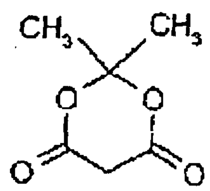

(II)

and an alcohol of formula (III)

$$R^3\text{-OH} \quad \text{(III)}$$

optionally in the presence of a diluent, under catalysis at temperatures within the range from 20 to 250°C, and that the malonic acid derivative of formula (IV) is subsequently reacted, optionally in the presence of a diluent, under catalysis at temperatures within the range from 20 to 250°C, with a salicylaldehyde of formula (V)

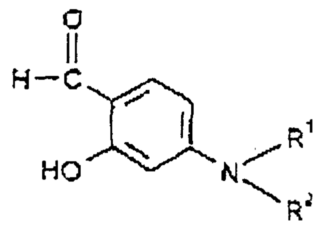

(V)

where $R^1$, $R^2$ and $R^3$ have the meanings given in claim 1, and
b) when Z represents

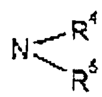

a salicylaldehyde of formula (V) is reacted with a secondary amine of formula (VI) and a malonic acid derivative of formula (VII)

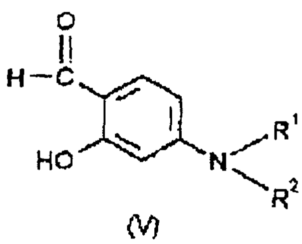

(V)

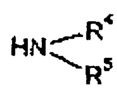

(VI)

$CH_2(COOR^6)_2$

(VII)

wherein

| | |
|---|---|
| $R^1$, $R^2$, $R^4$ and $R^5$ | have the meanings given above, and |
| $R^6$ | represents a $C_1$-$C_6$ alkyl, |

optionally in the presence of a diluent, at temperatures from 50 to 250°C.

**4.** The use of coumarin derivatives of formula (I) according to claim 1 for the production of polymers with luminophors on their side chains.

**5.** Coumarin derivatives of formula (I) according to claim 1:

3-(6-hydroxyhexoxycabonyl)-7-diethylaminocoumarin,
3-[N-hydroxyethyl-N-methyl)-amino-carbonyl]-7-diethylamino-coumarin.

**Revendications**

**1.** Dérivés de la coumarine répondant à la formule (I)

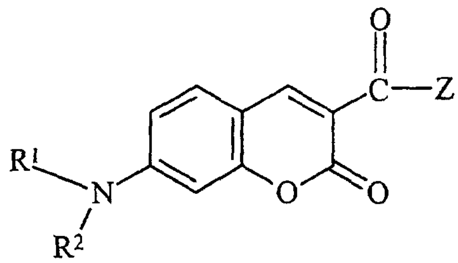

(I)

dans laquelle

$R^1$ et $R^2$ représentent chacun, indépendamment l'un de l'autre, un groupe alkyle en $C_1$-$C_{16}$ portant éventuellement des substituants (alcoxy en $C_1$-$C_4$)carbonyle, un groupe phényle, naphtyle, phényl-alkyle en $C_1$-$C_4$ ou naphtyl-alkyle en $C_1$-$C_4$, chacun d'eux non substitué ou portant des substituants alkyle en $C_1C_4$, (alcoxy en $C_1$-$C_4$)carbonyle, chloro et/ou bromo, ou bien

Z représente $OR^3$ ou $NR^4R^5$, $R^3$ représentant un groupe alkyle en $C_1$-$C_{16}$, phényle, naphtyle, phényl-alkyle en $C_1$-$C_4$ ou naphtyl-alkyle en $C_1$-$C_4$, chacun d'eux portant au moins un substituant hydroxy, et les cycles aromatiques pouvant en outre porter des substituants halogéno, alkyle en $C_1$-$C_6$, alcoxy en $C_1$-$C_6$,

$R^4$ et $R^5$ représentent chacun, indépendamment l'un de l'autre, un groupe alkyle en $C_1$-$C_{16}$, phényle, naphtyle, phényl-alkyle en $C_1$-$C_4$ ou naphtyl-alkyle en $C_1$-$C_4$ chacun d'eux portant éventuellement des substituants hydroxy, l'un des groupes $R^4$ ou $R^5$ portant un groupe hydroxy, et les cycles aromatiques pouvant en outre porter des substituants halogéno, alkyle en $C_1$-$C_6$, alcoxy en $C_1$-$C_6$, les chaînes carbonées aliphatiques, par exemple les parties alkyle, alcoxy, alkylamino, aralkyle de $R^1$, $R^2$, $R^3$, $R^4$ et $R^5$, pouvant être interrompues par un ou plusieurs, de préférence un ou deux hétéroatomes choisis parmi l'oxygène, l'azote et le soufre et/ou par un ou plusieurs, de préférence un ou deux cycles phénylène, lesquels peuvent porter des substituants alkyle en $C_1$-$C_4$ et/ou halogéno.

**2.** Dérivés de la coumarine de formule (I) de la revendication 1, **caractérisés en ce que**

$R^1$ et $R^2$ représentent des groupes alkyle en $C_1$-$C_6$ ou phényle portant éventuellement des substituants hydroxy, amino ou carboxy, ou bien

$R^1$ et $R^2$ : forment ensemble et avec l'atome d'azote auquel ils sont reliés un cycle morpholine, pipéridine, pyrrolidine ou pipérazine, lequel peut porter un ou deux substituants choisis parmi les groupes méthyle, éthyle et phényle,

$R^3$ représente un groupe alkyle en $C_1$-$C_{12}$ portant un substituant hydroxy,

$R^4$ et $R^5$ représentent chacun, indépendamment l'un de l'autre, un groupe alkyle en $C_1$-$C_{12}$ portant éventuellement des substituants hydroxy, l'un au moins des groupes $R^4$ et $R^5$ portant un groupe hydroxy.

**3.** Procédé de préparation des dérivés de la coumarine de formule (I) de la revendication 1 selon lequel

a) dans le cas où Z représente $-OR^3$, on prépare à partir de l'acide de Meldrum de formule (II)

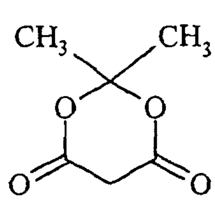

(II)

et d'un alcool de formule (III)

$R^3$-OH (III)

éventuellement en présence d'un diluant et avec catalyse, à des températures dans l'intervalle de 20 à 250°C, le dérivé de l'acide malonique répondant à la formule (IV)

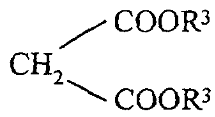

(IV)

qu'on fait ensuite réagir avec un aldéhyde salicylique de formule (V)

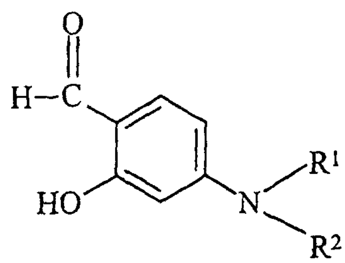

(V)

dans laquelle $R^1$, $R^2$, $R^3$ ont les significations indiquées dans la revendication 1,
éventuellement en présence d'un diluant, avec catalyse, à des températures de 50 à 250°C, et
b) dans le cas où Z représente

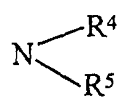

on fait réagir un aldéhyde salicylique de formule (V), une amine secondaire de formule (VI) et un dérivé de l'acide malonique répondant à la formule (VII)

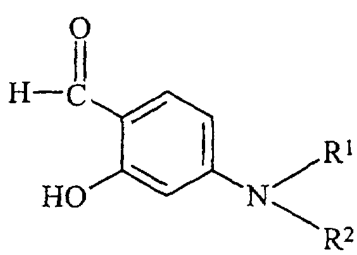

(V)

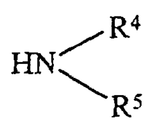

(VI)

$CH_2(COOR^6)_2$

(VII)

dans lesquelles

$R^1$, $R^2$, $R^4$ et $R^5$ ont les significations indiquées ci-dessus et
$R^6$ représente un groupe alkyle en $C_1$-$C_6$,

éventuellement en présence d'un diluant et à des températures de 50 à 250°C.

**4.** Utilisation des dérivés de la coumarine de formule (I) de la revendication 1, pour la préparation de polymères

contenant des luminophores dans les chaînes latérales.

5. Dérivés de la coumarine de formule (I) de la revendication 1 :

la 3-(6-hydroxyhexoxycarbonyl)-7-diéthylamino-coumarine,
la 3-[(6-N-hydroxyéthyl-N-méthyl)-aminocabonyl]-7-diéthylamino-coumarine.